(19) Europäisches Patentamt
European Patent Office
Office européen des brevets

(11) Numéro de publication : **0 321 349 B1**

# (12) FASCICULE DE BREVET EUROPEEN

(45) Date de publication du fascicule du brevet : **15.04.92 Bulletin 92/16**

(51) Int. Cl.$^5$ : **C07D 333/20**

(21) Numéro de dépôt : **88403198.0**

(22) Date de dépôt : **15.12.88**

## (54) Procédé de préparation de la N-(chloro-2 benzyl) (thiényl-2)-2 éthylamine.

(30) Priorité : **18.12.87 FR 8717755**

(43) Date de publication de la demande : **21.06.89 Bulletin 89/25**

(45) Mention de la délivrance du brevet : **15.04.92 Bulletin 92/16**

(84) Etats contractants désignés : **AT BE CH DE ES FR GB GR IT LI LU NL SE**

(56) Documents cités : **EP-A- 0 274 324**
**FR-A- 2 300 090**

(56) Documents cités : **JOURNAL OF THE AMERICAN CHEMICAL SOCIETY, vol. 76, no. 17, 9 septembre 1954, pages 4466-4468, American Chemical Society, US; E. CAMPAIGNE et al.: "3-Substituted thiophenes. VIII. 3-Thienylalkylamines"**

(73) Titulaire : **SANOFI**
**40, Avenue George V**
**F-75008 Paris (FR)**

(72) Inventeur : **Radisson, Joel**
**10 Avenue Winston Churchill**
**F-31100 Toulouse (FR)**

(74) Mandataire : **Varady, Peter et al**
**Cabinet Lavoix 2, Place d'Estienne d'Orves**
**F-75441 Paris Cedex 09 (FR)**

EP 0 321 349 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de la N-(chloro-2 benzyl) (thiényl-2)-2 éthylamine à partir du thiophène-2 acétonitrile.

Cette amine secondaire a été décrite pour la première fois dans le brevet FR-A-2 300 090 comme intermédiaire de synthèse du composé de formule :

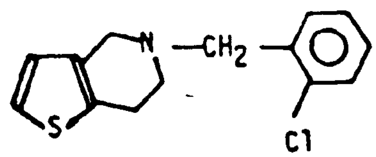

antiagrégant plaquettaire, dont la dénomination commune internationale est ticlopidine.

Le procédé de préparation de la N-(chloro-2 benzyl) (thiényl-2)-2 éthylamine, de formule :

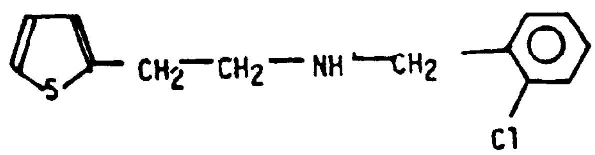

I

décrit dans le brevet FR-A-2 300 090 comprend la réaction de la chloro-2 benzylamine sur le benzènesulfonate de (thiényl-2)-2 éthyle selon le schéma réactionnel :

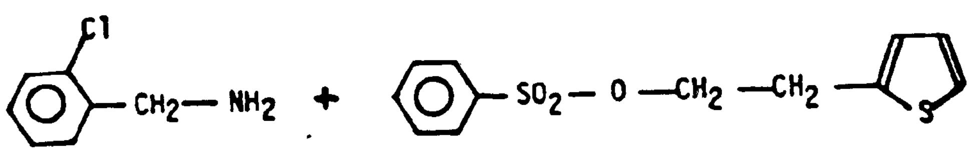

L'amine ci-dessus est disponible en quantités industrielles, mais le sulfonate doit être préparé à partir du (thiényl-2-)-2 éthanol, obtenu par réaction de l'oxyde d'éthylène sur l'organolithien :

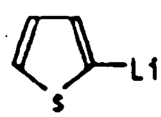

Bien que les rendements des réactions successives soient satisfaisants, l'ensemble du procédé est coûteux, d'autant plus qu'il implique de travailler, pour la première étape, en milieu rigoureusement anhydre.

EP-A- 274 324 décrit la synthèse de la di[(thiényl-2)-2éthyl]amine par hydrogènation catalytique du thienyl-2 acétonitrile.

On a maitenant trouvé, est c'est l'objet de l'invention, que le composé de formule I pouvait être préparé avec de bons rendements par réaction du thiophène-2 acétonitrile sur la chloro-2 benzylamine, en présence d'hydrogène et d'un catalyseur d'hydrogénation, selon le schéma réactionnel :

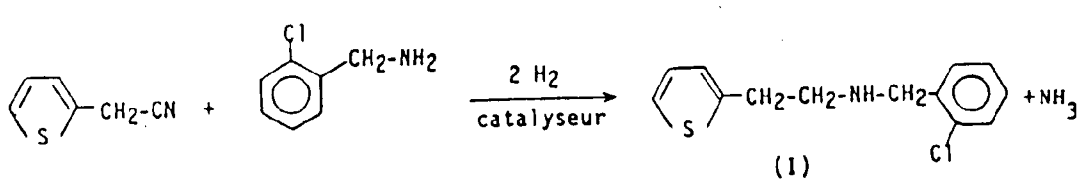


(I)

Le thiophène-2 acétonitrile et la chloro-2 benzylamine étant des produits commerciaux, l'accès au composé de formule (I) est rendu plus direct.

Le catalyseur utilisé est un catalyseur d'hydrogénation classique. Ce sera de préférence le nickel de Raney ou le palladium déposé sur un support inerte poreux, tel que le charbon actif.

La pression au cours de l'hydrogénation est habituellement comprise entre 10 et 100 bars (entre 1 et 10 MPa), de préférence entre 25 et 70 bars (entre 2,5 et 7 MPa).

La température de réaction est habituellement inférieure à 80°C, de préférence entre 40 et 60°C.

La réaction peut être effectuée sans solvant, ou dans un solvant organique inerte qui dissolve les réactifs et l'hydrogène, comme les alcools, de préférence le méthanol.

Le rapport molaire des réactifs :

$$\frac{\text{chloro-2 benzylamine}}{\text{thiophène-2 acétonitrile}}$$

peut être compris entre 1 et 5,
de préférence entre 1 et 2 dans le cas du palladium comme catalyseur, et de préférence entre 2 et 4 dans le cas du nickel de Raney.

Les réactifs peuvent être mélangés avant hydrogénation, ou bien le thiophène-2 acétonitrile peut être introduit progressivement sur la chloro-2 benzylamine en présence du catalyseur et de l'hydrogène.

La N-(chloro-2 benzyl) (thiényl-2)-2 éthylamine peut être isolée du mélange réactionnel par distillation sous pression réduite ou par précipitation de l'un de ses sels, comme le chlorhydrate.

L'excès de chloro-2 benzylamine peut être récupéré, soit par distillation sous pression réduite à partir du milieu réactionnel, soit sous forme de sel, après séparation du sel de (I), compte tenu de leurs différentes solubilités en milieu aqueux. Notamment dans un milieu constitué d'éther isopropylique et d'eau, le chlorhydrate de (I) précipite, tandis que le chlorhydrate de la chloro-2 benzylamine reste en phase aqueuse.

Les exemples suivants illustrent l'invention.

## Exemple 1

Dans un réacteur d'hydrogénation de 3500 ml, on introduit 100 g (0,81 mole) de thiophène-2 acétonitrile, 350 g (2,473 moles) de chloro-2 benzylamine et 25 g de nickel de Raney.

Le volume est ajusté à 1500 ml à l'aide de méthanol et l'ensemble est hydrogéné à 50° C sous une pression de 35 bars ± 5 bars (3,5 ± 0,5 MPa).

Lorsque la quantité théorique d'hydrogène a été absorbée, le catalyseur est filtré, le solvant évaporé et le concentrat distillé sous une pression de 0,3 mm de mercure (40 Pa).

La chloro-2 benzylamine en excès distille entre 60 et 65°C sous cette pression, puis la N-(chloro-2 benzyl) (thiényl-2)-2 éthylamine entre 120 et 130°C. On obtient ainsi 150 g de produit attendu, ce qui correspond à un rendement de 74,5% par rapport au thiophène-2 acétonitrile mis en oeuvre.

L'analyse centésimale et le spectre infra-rouge du produit isolé sont conformes.

## Exemple 2

Dans un réacteur d'hydrogénation de 500 ml, on introduit 20 g (0,16 mole) de thiophène-2 acétonitrile, 56,8 g (0,40 mole) de chloro-2 benzylamine, 8 g de nickel de Raney et 120 ml de méthanol. L'ensemble est hydrogéné à 50°C sous une pression de 35 bars ± 5 bars (3,5 ± 0,5 MPa).

Lorsque la quantité théorique d'hydrogène a été absorbée, le catalyseur est filtré et le solvant chassé sous vide.

Le concentrat est repris par 200 ml d'éther isopropylique et est agité 1 h à 0°C avec 100 ml d'acide chlorhydrique 6N. Le produit qui a cristallisé est filtré, lavé à l'eau puis à l'éther isopropylique et à l'acétone et est séché à poids constant à 50°C. On obtient 35,5 g de chlorhydrate de N-(chloro-2 benzyl) (thiényl-2)-2 éthylamine. P.F. = 145-147°C.

Spectre (infra-rouge) :
3330 $cm^{-1}$ : ν NH
3150 $cm^{-1}$ : ν CH (thiopène)
3060 $cm^{-1}$ : ν CH (benzène)

2940 $cm^{-1}$ : $\nu$ $CH_2$
2850-2200 $cm^{-1}$ : $\nu$ $>NH^+$
1590 et 1480 $cm^{-1}$ : C-C (benzène)
1450 $cm^{-1}$ : C-C (thiophène)
Spectre RMN : conforme.
765 $cm^{-1}$ :

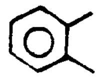

700 $cm^{-1}$ :

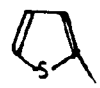

L'excès de chloro-2 benzylamine est extrait par l'éther isopropylique de la solution aqueuse acide après son alcalinisation. Deux extractions volume à volume et une distillation sous vide permettent de récupérer 29 g de chloro-2 benzylamine.

Exemple 3

Dans un hydrogénateur de 350 ml sont introduits 50 g de thiophène-2 acétonitrile, 280 g de chloro-2 benzylamine, 25 g de nickel de Raney.

Le mélange est hydrogéné à 50°C sous une pression de 35 ± 5 bars (3,5 ± 0,5 MPa) jusqu'à absorption de la quantité théorique d'hydrogène.

Le catalyseur est filtré, lavé au méthanol. Le filtrat est concentré puis distillé sous une pression de 0,3 mm de mercure (40 Pa), comme dans l'exemple 1.

On obtient ainsi 65 g de N-(chloro-2 benzyl) (thiényl-2)-2 éthylamine.

Rendement : 64,5 % par rapport au thiophène-2 acétonitrile mis en oeuvre.

Spectre I.R. et analyse centésimale conformes.

Exemple 4

Dans un hydrogénateur de 3,5 l sont introduits 20 g de nickel de Raney, 280 g (1,95 mole) de chloro-2 benzylamine et 500 ml de méthanol. La température est amenée à 50°C et la pression d'hydrogène à 25 bars (2,5 MPa).

On introduit alors à l'aide d'une pompe doseuse 100 g (0,80 mole) de thiophène-2 acétonitrile dilué dans 900 ml de méthanol. L'introduction est effectuée en 5 h.

Lorsque l'absorption d'hydrogène a cessé, le catalyseur est filtré et lavé au méthanol. Le méthanol est évaporé sous pression réduite et le concentrat est traité comme dans l'exemple 1. On obtient ainsi 152 g de produit attendu.

Rendement : 75,5 % par rapport au thiophène-2 acétonitrile mis en oeuvre.

Exemple 5

Dans un réacteur d'hydrogénation de 500 ml, on introduit 20 g (0,16 mole) de thiophène-2 acétonitrile, 45 g (0,32 mole) de chloro-2 benzylamine, 5g de palladium sur charbon à 10% et 130 ml de méthanol.

L'hydrogénation est conduite à 50°C sous une pression d'hydrogène de 60 ± 10 bars (6 ± 1 MPa).

Dès que l'absorption d'hydrogène a cessé, le catalyseur est filtré et le solvant évaporé sous vide.

Le concentrat est repris par 200 ml d'éther isopropylique et est agité 1h avec 100 ml d'acide chlorhydrique 6N.

Le produit qui a cristallisé est filtré, lavé à l'eau puis à l'éther isopropylique et à l'acétone, et est séché à poids constant à 50°C.

On obtient ainsi 22,9 g de chlorhydrate de N-(chloro-2 benzyl) (thiényl-2)-2 éthylamine.

Rendement par rapport au thiophène-2 acétonitrile consommé : 80,0%. Spectre I.R. identique à celui du

chlorhydrate obtenu à l'exemple 2.

Par évaporation de la première phase éthérée, on obtient 7,8 g de thiophène-2 acétonitrile qui peut être recyclé.

Par alcalinisation de la phase aqueuse et extraction à l'éther isopropylique, on obtient 29,9 g de chloro-2 benzylamine qui peut être recyclée.

Exemple 6

Dans un réacteur d'hydrogénation de 500 ml, on introduit 20 g (0,16 mole) de thiophène-2 acétonitrile, 25 g (0,176 mole) de chloro-2 benzylamine, 5g de palladium sur charbon à 10% et 150 ml de méthanol.

L'hydrogénation est conduite à 50°C sous une pression d'hydrogène de 60 ± 10 bars (6 ± 1 MPa).

Le mélange réactionnel est ensuite traité comme dans l'exemple n° 4. On obtient ainsi 16,7 g de chlorhydrate de N-(chloro-2 benzyl) thiényl-2)-2 éthylamine.

Par évaporation de la première phase éthérée, on obtient 10,0 g de thiophène-2 acétonitrile qui peut être recyclé.

Rendement par rapport au thiophène-2 acétonitrile consommé : 82,5 %.

## Revendications

1. Procédé de préparation de la N-(chloro-2 benzyl) (thiényl-2)-2 éthylamine, caractérisé en ce que l'on fait réagir le thiophène-2 acétonitrile sur la chloro-2 benzylamine en présence d'hydrogène et d'un catalyseur d'hydrogénation.

2. Procédé selon la revendication 1, caractérisé en ce que la pression d'hydrogène est comprise entre 1 et 10 MPa.

3. Procédé selon la revendication 2, caractérisé en ce que la pression d'hydrogène est comprise entre 2,5 et 7 MPa.

4. Procédé selon l'une des revendications 1 à 3, caractérisé en ce que la réaction est effectuée à une température inférieure à 80°C.

5. Procédé selon la revendication 4, caractérisé en ce que la température est comprise entre 40 et 60°C.

6. Procédé selon l'une des revendications 1 à 5, caractérisé en ce que la réaction est effectuée dans un alcool comme solvant.

7. Procédé selon l'une des revendications 1 à 6, caractérisé en ce que le rapport molaire de la chloro-2 benzylamine au thiophène-2 acétonitrile est compris entre 1 et 5.

8. Procédé selon la revendication 7, caractérisé en ce que le catalyseur est le nickel de Raney et le rapport molaire desdits réactifs est compris entre 2 et 4.

9. Procédé selon la revendication 7, caractérisé en ce que le catalyseur est le palladium et le rapport molaire desdits réactifs est compris entre 1 et 2.

## Claims

1. A process for preparing N- (2-chloro-benzyl) -2-(2-thienyl) ethylamine, characterised in that 2-thiophene acetonitrile is reacted with 2-chlorobenzylamine and hydrogen in the presence of a hydrogenation catalyst.

2. A process according to Claim 1, characterised in that the hydrogen pressure ranges from 1 to 10 MPa.

3. A process according to Claim 2, characterised in that the hydrogen pressure ranges from 2.5 to 7 MPa.

4. A process according to Claim 1, characterised in that the reaction is carried out at a temperature lower than 80°C.

5. A process according to Claim 4, characterised in that the temperature ranges from 40 to 60°C.

6. A process according to Claim 1, characterised in that the reaction is carried out in an alcohol as solvent.

7. A process according to Claim 1, characterised in that the molar ratio of the 2-chlorobenzylamine to the 2-thiophene acetonitrile ranges from 1 to 5.

8. A process according to Claim 7, characterised in that the catalyst is Raney nickel and the molar ratio of said reagents ranges from 2 to 4.

9. A process according to Claim 7, characterised in the catalyst is palladium and the molar ratio of said reagents ranges from 1 to 2.

## Patentansprüche

1. Verfahren zur Herstellung von N-(2-chlorbenzyl)-2-(2-thienyl)-ethylamin, dadurch **gekennzeichnet**, daß man 2-Thienylacetonitril mit 2-Chlorbenzylamin in Gegenwart von Wasserstoff und einem Hydrierungskatalysator umsetzt.

2. Verfahren nach Anspruch 1, dadurch gekennzeichnet, daß der Wasserstoffdruck zwischen 1 und 10 MPa liegt.

3. Verfahren nach Anspruch 2, dadurch gekennzeichnet, daß der Wasserstoffdruck zwischen 2,5 und 7 MPa liegt.

4. Verfahren nach einem der Ansprüche 1 bis 3, dadurch gekennzeichnet, daß die Reaktion bei einer Temperatur unter 80 °C durchgeführt wird.

5. Verfahren nach Anspruch 4, dadurch gekennzeichnet, daß die Temperatur zwischen 40 und 60 °C liegt.

6. Verfahren nach einem der Ansprüche 1 bis 5, dadurch gekennzeichnet, daß die Reaktion in einem Alkohol als Lösungsmittel durchgeführt wird.

7. Verfahren nach einem der Ansprüche 1 bis 6, dadurch gekennzeichnet, daß das molare Verhältnis des 2-Chlorbenzylamins zum 2-Thienylacetonitril zwischen 1 und 5 liegt.

8. Verfahren nach Anspruch 7, dadurch gekennzeichnet, daß der Katalysator Raney-Nickel ist und das molare Verhältnis der Reagenzien zwischen 2 und 4 liegt.

9. Verfahren nach Anspruch 7, dadurch gekennzeichnet, daß der Katalysator Palladium ist und das molare Verhältnis der Reagenzien zwischen 1 und 2 liegt.